# EUROPÄISCHE PATENTANMELDUNG

(11) **EP 2 127 633 A1**
(43) Veröffentlichungstag der Anmeldung: **02.12.2009**
(21) Anmeldenummer: 09155885.8
(22) Anmeldetag: 23.03.2009
(51) Int. Cl.: A61K 8/06, A61K 8/25, A61K 8/35, A61K 8/49, A61K 8/895, A61Q 17/04

(54) **Sonnenschutzzusammensetzungen**

(30) Priorität: 28.05.2008 DE 102008025576
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE)

(57) **Zusammenfassung**

Gegenstand der vorliegenden Anmeldung sind kosmetische Zusammensetzungen mit verbessertem Lichtschutzfaktor, enthaltend mindestens ein Alkyl- und/oder Alkoxysubstituiertes Dibenzoylmethanderivat, Polysilicone-15, mindestens einen wasserlöslichen UV-Filter, umfassend ein Derivat der Benzimidazolsulfonsäure, sowie 0,2 - 5 Gew.-% Siliciumdioxid-Partikel, die nicht organisch oder anorganisch modifiziert sind und keine organische oder anorganische Beschichtung aufweisen.

## Beschreibung

Die vorliegende Erfindung betrifft kosmetische und dermatologische Zusammensetzungen zum Schutz von Haut und Haar gegen UV-Strahlung.

Die hautschädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.

Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und

Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahresund tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

### Aufgabe

Die Zusammensetzungen des Standes der Technik sind weiter verbesserbar. Insbesondere weisen Zusammensetzungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren. Ein weiterer Nachteil des Standes der Technik ist, dass übliche Lichtschutzformulierungen einen meist klebrigen Film auf der Haut hinterlassen. Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig verbesserte sensorische Eigenschaften aufweisen. Die erfindungsgemäßen Zusammensetzungen sollen ferner gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

### Stand der Technik

US 6258857 offenbart gemäß Anspruch 1 eine Flüssig-Flüssig-Dispersion, enthaltend synthetische Harzpartikel sowie feinteilige poröse anorganische Partikel, die mit einer Chemikalie beladen sind, nicht-hohl oder hohl sein können, eine Teilchengröße von 0,5 - 50 µm, eine mittlere Teilchengröße von 0,5 - 30 µm, Durchmesser der Oberflächenporen von 20 bis 150 Å, eine spezifische Oberfläche von 20 - 800 m²/g und ein Porenvolumen von 0,01 - 1,50 ml/g aufweisen. Gemäß Spalte 5, Zeile 26, und Spalte 7, Zeile 66 - Spalte 8, Zeile 4, kann der als "chemical" bezeichnete Wirkstoff aus einer langen Liste von kosmetischen Wirkstoffen ausgewählt sein, darunter anorganische UV-Filter. Entsprechende Formulierungsbeispiele sind nicht realisiert.

EP 796612 A1 offenbart kosmetische Zusammensetzungen, enthaltend eine Fettphase, einen partikelförmigen, also ungelösten, organischen oder anorganischen UV-Filter sowie einen Füllstoff, wobei beide Substanzen in einem bestimmten Volumenverhältnis zueinander vorliegen, zur Steigerung des Lichtschutzfaktors. Unter den geeigneten Füllstoffen sind sowohl sphärische Silica-Partikel als auch lamellare Mica-Partikel offenbart. Ein Hinweis auf die Steigerung des Lichtschutzfaktors gelöster UV-Filter durch sphärische Silica-Partikel findet sich nicht.

EP 1421931 A2 offenbart kosmetische Sonnenschutzzusammensetzungen, enthaltend sphärische Silica-Partikel zur Steigerung des Lichtschutzfaktors öl- und wasserlöslicher UV-Filter. Diese Schrift gibt dem Fachmann allerdings keinen Hinweis darauf, dass mit der vorliegend beanspruchten UV-Filterkombination sogar eine Verdopplung des Lichtschutzfaktors erzielt werden kann. Offenbart ist eine Sonnenschutzzusammensetzung, die in einem O/W-Emulsionsträger 1 Gew.-% poröse Silica-Beads sowie eine Filterkombination aus 10 Gew.-% Octocrylene, 1 Gew.-% Ethylhexyltriazone, 3 Gew.-% Drometrizole trisiloxane, 3 Gew.-% Butylmethoxydibenzoylmethan, 0,5 Gew.-% Terephthalylidene dicamphor sulfonic acid und 5 Gew.-% Titandioxid enthält. Gegenüber der gleichen Zusammensetzung ohne poröse Silica-Beads wird der Lichtschutzfaktor von 15,5 auf 21,5 verbessert.

Gegenstand der vorliegenden Anmeldung sind kosmetische Zusammensetzung, enthaltend mindestens einen öllöslichen UV-Filter, umfassend mindestens ein Alkyl- und/oder Alkoxy-substituiertes Dibenzoylmethanderivat, weiterhin Polysilicone-15 sowie mindestens einen wasserlöslichen UV-Filter, umfassend mindestens ein Derivat der Benzimidazolsulfonsäure, sowie 0,2 - 5 Gew.-% Siliciumdioxid-Partikel, die nicht organisch oder anorganisch modifiziert sind und keine organische oder anorganische Beschichtung aufweisen.

Gegenstand der Erfindung ist ferner die Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung.

Bevorzugte Ausführungsformen der vorliegenden Erfindung ergeben sich aus den abhängigen Patentansprüchen.

Die erfindungsgemäßen Zusammensetzungen stellen in jeglicher Hinsicht überaus befriedigende Präparate dar, die im Hinblick auf die weiteren Bestandteile nicht auf eine spezielle Auswahl dieser weiteren Bestandteile begrenzt sind. Dementsprechend eignen sie sich ganz besonders, um als Grundlage für Zusammensetzungsformen mit vielfältigen Anwendungszwecken zu dienen. Die erfindungsgemäßen Zusammensetzungen zeigen überraschenderweise synergistisch verbesserte sensorische und kosmetische Eigenschaften, wie beispielsweise die gleichmäßige Verteilbarkeit auf der Haut oder das Einzugsvermögen in die Haut, und zeichnen sich gleichzeitig durch eine hohe Lichtschutzeffektivität bei gleichzeitig hervorragenden Hautpflegedaten aus.

Ein Maß für die UV-Schutzleistung stellt im Sinne der vorliegenden Erfindung beispielsweise der Lichtschutzfaktor (LSF bzw. SPF) dar.

Die kosmetischen und dermatologischen Zusammensetzungen im Sinne der vorliegenden Erfindung hinterlassen auf der Haut keinen schmierigen oder klebrigen Eindruck und sind ausgezeichnet hautverträglich.

### a) Alkyl- und/oder Alkoxy-substituiertes Dibenzoylmethanderivat

Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer AlkoxyGruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als PARSOL^{®} 1789 von DSM oder als Eusolex^{®} 9020 von Merck KGaA erhältlich ist.

### b) Polysilicone-15

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

### c) Derivat der Benzimidazolsulfonsäure

Bevorzugte UV-Filtersubstanz(en) der Komponente (c) der erfindungsgemäßen Zusammensetzungen ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere das Phenylen-1,4-bis-(2-benzimidazyl)-3,3'-5,5'-tetrasulfonsäure-bisnatriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte UV-Filtersubstanz(en) der Komponente (c) der erfindungsgemäßen Zusammensetzungen ist bzw. sind die 2-Phenylbenzimidazol-5-sulfonsäure (UV-B) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere aber die 2-Phenylbenzimidazol-5-sulfonsäure selbst mit der INCI-Bezeichnung "Phenylbenzimidazole sulfonic acid" (CAS.-Nr. 27503-81-7), die beispielsweise unter den Handelsnamen Neo Heliopan Hydro von Symrise oder Eusolex 232 von Merck KGaA erhältlich ist.

In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist.

In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.

Erfindungsgemäß besonders bevorzugte Lichtschutzfilter-Kombinationen (a), (b) und (c) umfassen 4-tert-Butyl-4'-Methoxydibenzoylmethan, Polysilicone-15 und 2-Phenylbenzimidazol-5-sulfonsäure. Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und Dinatriumphenylbenzimidazoltetrasulfonat.

Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und ein 2-Phenylbenzimidazol-5-sulfonsäure-Arginin-Salz.

Die oben genannten UV-Filtersubstanzen der Komponente (a) sind bevorzugt in einer Gesamtmenge von 0,5 Gew.-% bis 20 Gew.-%, besonders bevorzugt 1 bis 15 Gew.-%, insbesondere 2 bis 10 Gew.-% und außerordentlich bevorzugt 3 - 5 Gew.-%, enthalten, jeweils bezogen auf das Gesamtgewicht der erfindungsgemäßen Zusammensetzungen.

Polysilicone-15, die oben genannte Komponente (b), ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.

Die oben genannten UV-Filtersubstanzen der Komponente (c) sind bevorzugt in einer Gesamtmenge von 0,5 Gew.-% bis 15 Gew.-%, besonders bevorzugt 1 bis 10 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.

Bevorzugte erfindungsgemäße Zusammensetzungen sind **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5), bevorzugt wie (0,9 - 1,2) : (0,9 - 1,2) : (0,9 - 1,2), besonders bevorzugt wie (1 -1,1): (1 - 1,1) : (1 - 1,1), enthalten sind.

Um den Lichtschutzfaktor weiter zu erhöhen, können die erfindungsgemäßen Zusammensetzungen bevorzugt mindestens eine weitere organische und/oder anorganische UV-Filtersubstanz enthalten. Weitere bevorzugte UV-Filtersubstanzen, die neben denen der Komponenten (a), (b) und (c) in den erfindungsgemäßen Zusammensetzungen eingesetzt werden, sind im Folgenden genannt.

### Triazinderivate

UV-Filtersubstanzen auf Basis von Triazinderivaten, die das nachfolgende Strukturmotiv 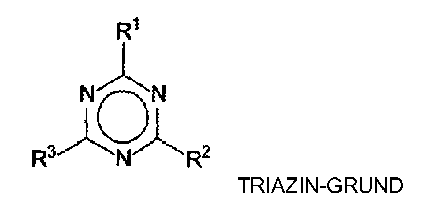 aufweisen, sind im Stand der Technik bekannt, beispielsweise aus EP 775698, EP 878469 und EP 1027881.

Hinsichtlich der C₃-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.

Hinsichtlich der C₃-Achse des Triazin-Grundkörpers symmetrische Triazinderivate sind bevorzugt solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen. Besonders bevorzugte derartige symmetrische Triazinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltriimino)-tris-benzoesäuretris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist. Erfindungsgemäß bevorzugte unsymmetrische Triazinderivate sind beispielsweise solche, die in EP 775698 offenbart sind: 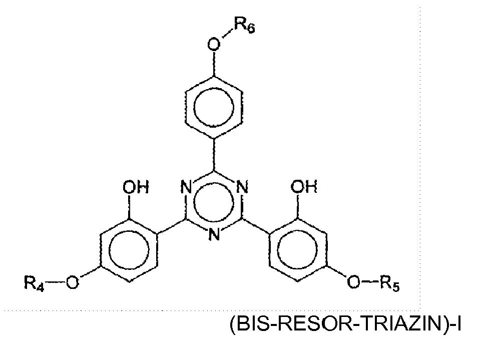 und/oder

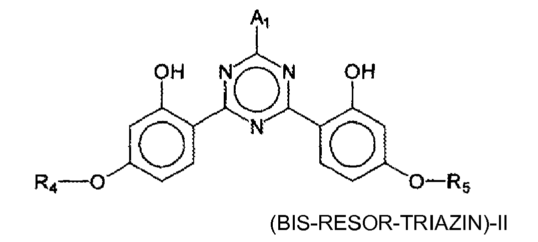

Alle in EP 775698 erwähnten so genannten Bis-Resorcinyltriazine, seien sie durch generische oder durch konkrete Formeln offenbart, sind vorteilhaft im Sinne der vorliegenden Erfindung.

Ganz besonders bevorzugt werden R₄ und R₅ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

A₁ stellt bevorzugt einen substituierten homo- oder heterocyclischen aromatischen Fünfring oder Sechsring dar.

Ganz besonders bevorzugte optionale UV-Filtersubstanzen sind ausgewählt aus unsymmetrisch substituierten s-Triazin-Verbindungen der allgemeinen Formel (BIS-RESOR-TRIAZIN)-I, in der R₆ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-I, in der R₄ und R₅ jeweils eine 2-Ethylhexyl-Gruppe und R₆ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere, erfindungsgemäß bevorzugte optionale unsymmetrisch substituierte s-TriazinVerbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = PhenylRest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist.

Weitere, erfindungsgemäß bevorzugte optionale UV-Filtersubstanzen auf Basis von s-Triazin-Verbindungen sind:
- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3 ,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Die s-Triazinderivate werden bevorzugt in die Ölphase der erfindungsgemäßen kosmetischen oder dermatologischen Zusammensetzungen eingearbeitet.

### Benzotriazole

Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 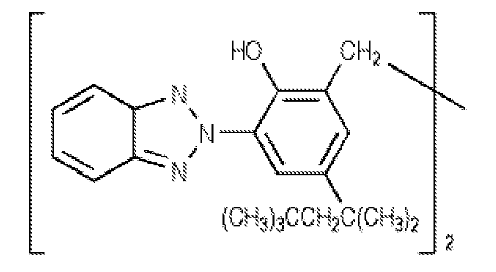 , unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (a), (b) und (c) in den erfindungsgemäßen Zusammensetzungen eingesetzt werden, sind im Folgenden genannt:
- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter der Warenbezeichnung Eusolex 6300 vertrieben wird;
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich).
- 4-Aminobenzoësäure-Derivate, bevorzugt 4-(Dimethylamino)-benzoësäure(2-ethylhexyl) - ester, 4-(Dimethylamino)benzoësäureamylester;
- 2-Aminobenzoësäure-Derivate
- Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)),
- Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat), Salicylsäurehomomenthylester (Homomenthylsalicylat, Homosalate).
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus bei der Firma BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)-ester,
- sowie an Polymere gebundene UV-Filter, die von Komponente (b) verschieden sind.
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine).
   Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimt-säure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Die Liste der genannten optionalen UV-Filtersubstanzen, die im Sinne der vorliegenden Erfindung eingesetzt werden können, soll selbstverständlich nicht limitierend sein.

Die Gesamtmenge an der mindestens einen optionalen organischen UV-Filtersubstanz in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung.

Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira.

Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Die erfindungsgemäßen kosmetischen und dermatologischen Zusammensetzungen enthalten als Komponente (d) der erfindungsgemäßen Kombination mindestens einen Feststoff, der aus Siliciumdioxid-Partikeln, die nicht organisch oder anorganisch modifiziert sind und keine organische oder anorganische Beschichtung aufweisen, ausgewählt ist, in einer Gesamtmenge von 0,2 - 5 Gew.-%, bezogen auf die gesamte Zusammensetzung.

Das Merkmal der Komponente (d), "keine organische oder anorganische Beschichtung", bezieht sich erfindungsgemäß auf den Zustand der Siliciumdioxid-Partikel, bevor sie mit den übrigen Bestandteilen der erfindungsgemäßen Zusammensetzungen vermischt werden. Nach dem Vermischen ist es durchaus möglich, dass einzelne Rezepturbestandteile an der Oberfläche der Siliciumdioxid-Partikel sorbiert oder sorbiert werden; das Merkmal "keine organische oder anorganische Beschichtung" bleibt davon unbenommen. Auch möglicherweise sorbierte Gase gelten erfindungsgemäß nicht als Beschichtung.

Eine erste erfindungsgemäß bevorzugte Ausführungsform ist **dadurch gekennzeichnet, dass** die Siliciumdioxid-Partikel (d) durch Kieselsäure-Fällung erhältlich sind.

Eine weitere erfindungsgemäß bevorzugte Ausführungsform ist **dadurch gekennzeichnet, dass** die Siliciumdioxid-Partikel (d) zu mehr als 80 Gew.-% sphärische Partikel umfassen.

Eine weitere erfindungsgemäß bevorzugte Ausführungsform ist **dadurch gekennzeichnet, dass** die Siliciumdioxid-Partikel (d) einen zahlenmittleren Partikeldurchmesser im Bereich von 2 - 15 µm, besonders bevorzugt im Bereich von 5 - 10 µm, aufweisen.

Eine weitere erfindungsgemäß bevorzugte Ausführungsform ist **dadurch gekennzeichnet, dass** die Siliciumdioxid-Partikel (d) eine Ölabsorptionskapazität von 0,7 - 1,5 cm³ flüssiges Paraffin pro Gramm trockenes Siliciumdioxid, aufweisen, gemessen nach DIN 53601.

Eine weitere erfindungsgemäß bevorzugte Ausführungsform ist **dadurch gekennzeichnet, dass** die Siliciumdioxid-Partikel (d) durch Kieselsäure-Fällung erhältlich sind und zu mehr als 80 Gew.-% sphärische Partikel umfassen.

Eine weitere erfindungsgemäß bevorzugte Ausführungsform ist **dadurch gekennzeichnet, dass** die Siliciumdioxid-Partikel (d) durch Kieselsäure-Fällung erhältlich sind und einen zahlenmittleren Partikeldurchmesser im Bereich von 2 - 15 µm, besonders bevorzugt im Bereich von 5 - 10 µm, aufweisen.

Eine weitere erfindungsgemäß bevorzugte Ausführungsform ist **dadurch gekennzeichnet, dass** die Siliciumdioxid-Partikel (d) durch Kieselsäure-Fällung erhältlich sind, zu mehr als 80 Gew.-% sphärische Partikel umfassen und einen zahlenmittleren Partikeldurchmesser im Bereich von 2 - 15 µm, besonders bevorzugt im Bereich von 5 - 10 µm, aufweisen.

Eine weitere erfindungsgemäß bevorzugte Ausführungsform ist **dadurch gekennzeichnet, dass** die Siliciumdioxid-Partikel (d) durch Kieselsäure-Fällung erhältlich sind, zu mehr als 80 Gew.-% sphärische Partikel umfassen, einen zahlenmittleren Partikeldurchmesser im Bereich von 2 - 15 µm, besonders bevorzugt im Bereich von 5 - 10 µm, und eine Ölabsorptionskapazität von 0,7 - 1,5 cm³ flüssiges Paraffin pro Gramm trockenes Siliciumdioxid, gemessen nach DIN 53601, aufweisen.

Bevorzugte Siliciumdioxid-Partikel (d) sind als Handelsprodukt SB-705 der Firma Miyoshi Kasei, erhältlich, ein sphärisches Kieselgel mit der INCI-Bezeichnung Silica, das einen zahlenmittleren Teilchendurchmesser von 5 - 6 µm und eine spezifische Oberfläche von etwa 600 m²/g aufweist. Weitere bevorzugte Siliciumdioxid-Partikel (d) sind als Handelsprodukt Aerosil von Evonik Degussa erhältlich: Aerosil 130, Aerosil 200, Aerosil 255, Aerosil 300 oder Aerosil 380. Weitere bevorzugte Siliciumdioxid-Partikel (d) sind ebenfalls als Handelsprodukte erhältlich: CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), Cosmedia Silc (Cognis), Neosil PC 50 S (Ineos Silicas), Sorbosil BFG 54 (Ineos Silicas), Sorbosil AC33 (Ineos Silicas), Sorbosil AC 35 (Ineos Silicas), Sorbosil AC 37 (Ineos Silicas), Sorbosil AC 39 (Ineos Silicas), Wacker HDK H 30 (Wacker-Chemie), Wacker HDK N 20 (Wacker-Chemie), Wacker HDK S 13 (Wacker-Chemie), Wacker HDK T 30 (Wacker-Chemie), Wacker HDK V 15 (Wacker-Chemie).

Die erfindungsgemäßen Zusammensetzungen sind **dadurch gekennzeichnet, dass** sie die Siliciumdioxid-Partikel (d) in einer Gesamtmenge von 0,2 - 5 Gew.-%, bevorzugt 1 - 4 Gew.-%, besonders bevorzugt 1,5 - 3,5 Gew.-%, außerordentlich bevorzugt 2 - 3 Gew.-% und weiter bevorzugt 2,5 - 2,8 Gew.-%, jeweils bezogen auf die gesamte Zusammensetzung, enthalten.

Um die haptischen Eigenschaften und/oder den Lichtschutzfaktor der erfindungsgemäßen Zusammensetzungen weiter zu verbessern, können diese bevorzugt mindestens einen weiteren teilchenförmigen Feststoff, der von Komponente (d) verschieden ist, enthalten. Weitere bevorzugte optionale teilchenförmige Feststoffe, die neben Komponente (d) in den erfindungsgemäßen Zusammensetzungen eingesetzt werden, sind im Folgenden genannt.

Bevorzugte optionale teilchenförmige Feststoffe sind ausgewählt aus färbenden, farbgebenden, mattierenden oder glanzgebenden Pigmenten. Bevorzugte Pigmente dieser Art können anorganisch oder organisch sein. Weitere bevorzugte Pigmente weisen einen zahlenmittleren Teilchendurchmesser von 0,1 - 200 µm, bevorzugt 0,5 - 100 µm, besonders bevorzugt 1 - 50 µm und außerordentlich bevorzugt 2 - 30 µm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Besonders bevorzugte Farbpigmente sind beispielsweise Titandioxid, Glimmer, Eisenoxide (z. B. Fe₂O₃, Fe₃O₄, FeO(OH)) und/oder Zinnoxid. Besonders bevorzugte Farbstoffe sind beispielsweise Carmin, Berliner Blau, Chromoxidgrün, Ultramarinblau und/oder Manganviolett.

Erfindungsgemäß bevorzugte Perlglanzpigmente sind ausgewählt aus natürlichen Perlglanzpigmenten, wie z. B. "Fischsilber" (Guanin/Hypoxanthin-Mischkristalle aus Fischschuppen) und "Perlmutt" (vermahlene Muschelschalen), monokristallinen Perlglanzpigmenten, wie z. B. Bismuthoxychlorid (BiOCl), und Schicht-Substrat Pigmenten, z. B. Glimmer / Metalloxid. Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere bevorzugt ist z. B. das unter der CIN 77163 aufgelistete Glanzpigment.

Erfindungsgemäß bevorzugt sind ferner die folgenden Perlglanzpigmentarten auf Basis von Metalloxid-beschichtetem Glimmer:

| Gruppe | Beschichtung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂/ Fe₂O₃ | Goldtöne |
| | TiO₂/ Cr₂O₃ | grün |
| | TiO₂/ Berliner Blau | tiefblau |
| | TiO₂/ Carmin | rot |

Erfindungsgemäß besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich außer Glimmer auch andere Substrate mit weiteren Metalloxiden beschichten, wie z. B. Silica und dergleichen mehr. Vorteilhaft sind z. B. mit TiO₂und Fe₂O₃ beschichtete SiO₂-Partikel ("Ronasphere LDP") von der Firma Merck, die sich besonders für die optische Reduktion feiner Fältchen eignen.

Es kann darüber hinaus erfindungsgemäß bevorzugt sein, gänzlich auf ein Substrat wie Glimmer zu verzichten. Besonders bevorzugt sind Perlglanzpigmente, die unter der Verwendung von SiO₂ hergestellt werden. Solche Pigmente, die auch zusätzlich goniochromatische Effekte haben können, sind z. B. unter dem Handelsnamen Sicopearl Fantastico bei der Firma BASF erhältlich.

Weiterhin bevorzugt sind Pigmente der Firma Engelhard / Mearl auf Basis von Calcium Natrium Borosilikat, die mit Titandioxid beschichtet sind. Diese sind unter dem Namen Reflecks erhältlich. Sie weisen durch ihrer Partikelgröße von 40 - 180 µm zusätzlich zu der Farbe einen Glitzereffekt auf.

Besonders vorteilhaft sind ferner auch Effektpigmente, die unter der Handelsbezeichnung Metasomes Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Weitere bevorzugte Pigmente sind ausgewählt aus farbigen und farblosen Pigmenten. Einige der im Folgenden genannten Pigmente dienen auch als UV-Absorber. Besonders bevorzugte farbige Pigmente sind ausgewählt aus den Eisenoxiden mit den Color Index-Nummern Cl 77491 (Eisenoxid rot), Cl 77492 (Eisenoxidhydrat gelb) und Cl 77499 (Eisenoxid schwarz), aus Cl 77891 (Titandioxid) und Ruß.

Besonders bevorzugte Pigmente sind die Handelsprodukte SUNPMMA-S und SUNSIL Tin 30 der Firma Sunjin Chemicals Co. mit einem zahlenmittleren Teilchendurchmesser von 5 - 10 µm bzw. 2 - 7 µm.

Besonders bevorzugte optionale anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere^{®} LDP der Firma Merck KGaA. Bei diesem Produkt handelt es sich um sphärische Siliciumdioxid-Partikel, die mit Titandioxid und Eisenoxid beschichtet sind. Ronasphere^{®} LDP weist einen zahlenmittleren Teilchendurchmesser von 4 - 7 µm auf. Weiterhin bevorzugt sind anorganisch beschichtete Glimmerpigmente, die keinen Perlglanz aufweisen. Weitere bevorzugte optionale anorganisch beschichtete anorganische Pigmente sind Glimmerpigmente, die mit Titandioxid in verschiedenen Schichtdicken beschichtet sind, beispielsweise die Produkte der Timiron^{®}-Serie von Rona/Merck KGaA, insbesondere Pigmente der Produktreihen Timiron^{®} MP, Timiron^{®} Super, Timiron^{®} Starlight und Timiron^{®} Silk. Die genannten Produkte weisen mittlere Teilchendurchmesser von 5 - 60 µm bzw. 10 - 60 µm bzw. 10 - 125 µm bzw. 5 - 25 µm auf. Ebenfalls bevorzugt sind Glimmerpartikel mit einer Beschichtung aus Titandioxid und

Eisenoxid, z. B. die Handelsprodukte Timiron^{®} MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona^{®}-Serie. Weitere bevorzugte Pigmente sind mit Kieselgel beschichtete Glimmerpigmente, z. B. das Handelsprodukt Micronasphere^{®} M. Weitere erfindungsgemäß bevorzugte Pigmente sind anorganisch beschichtete anorganische Pigmente, deren Beschichtung einen Anteil von 0,1 - 1 Gew.% Zinnoxid aufweist.

Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Très BN^{®} UHP 1106 von Carborundum), mit einem Gemisch aus Dimethylpolysiloxan und Silicagel (Simethicone) und Aluminiumoxidhydrat (Alumina) beschichtetes Titandioxid (Eusolex^{®} T 2000 von Merck), mit Octylsilanol beschichtetes Titandioxid oder sphärische Polyalkylsesquisiloxan-Partikel (Aerosil^{®} R972 von Degussa).

Weitere erfindungsgemäß besonders bevorzugte Zusammensetzungen sind **dadurch gekennzeichnet, dass** sie mindestens ein färbendes, farbgebendes, mattierendes oder glanzgebendes Pigment in einer Gesamtmenge von 0,1 Gew.-% bis 30 Gew.-%, bevorzugt 0,5 bis 15 Gew.-%, besonders bevorzugt 1,0 bis 10 Gew.-% und außerordentlich bevorzugt 2 - 5 Gew.-%, enthalten, jeweils bezogen auf das Gesamtgewicht der erfindungsgemäßen Zusammensetzung.

Weitere bevorzugte optionale teilchenförmige Feststoffe sind die durch Hydrolyse und Kondensationsreaktionen von Methyltrimethoxysilane erhältlichen Polymethylsilsesquioxane, die sphärisch sein können (insbesondere Aerosil^{®} R 972 und Aerosil^{®} 200 V von Evonik Degussa), und deren Teilchengrößenverteilung durch die Herstellung gesteuert werden kann. Bevorzugte Polymethylsilsesquioxane werden beispielsweise unter den Handelsnamen Tospearl 2000 B und Tospearl 145A von Momentive Performance Materials, AEC Silicone Resin Spheres von A & E Connock sowie Wacker-Belsil PMS MK von der Wacker-Chemie angeboten.

Weitere bevorzugte optionale teilchenförmige Feststoffe sind ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten wie Natriumstärkeoctenylsuccinat, Aluminiumstärkeoctenylsuccinat oder Distarch Phosphate (z. B. Corn PO4 (Agrana Stärke), Corn PO4 (Tri-K)), Maisstärke Zea Mays (Amidon De Mais MST (Wackherr), Argo Brand Corn Starch (Corn Products), Pure-Dent (Grain Processing), Purity 21C (National Starch)), Cellulose, insbesondere mikrokristalliner Cellulose, und Cellulosederivaten, insbesondere Celluloseethern, wie Ethylcellulose, Hydroxyethylcellulose, Hydroxypropylcellulose oder Hydroxypropylmethylcellulose, Kieselgelen, Talkum, Kaolin, Silicaten, insbesondere Schichtsilicate und unter diesen insbesondere Montmorillonit, Kaolinit, IIIit, Beidellit, Nontronit, Saponit, Hectorit, Bentoniten, Smectit, Magnesiumaluminiumsilikaten, insbesondere Talkum, Carbonaten, wie z. B. Magnesiumcarbonat (MgCO₃) und Calciumcarbonat (CaCO₃), .Bornitrid, Lactoglobulinderivaten, z. B. Natrium-C₈-₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Bevorzugte Polymerpulver auf Basis eines Polymethacrylat-Copolymers sind z. B. als Handelsprodukt Polytrap^{®} 6603 (Dow Corning) erhältlich. Andere bevorzugte Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethylmethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (Microthene^{®} von Omya oder ACCUREL^{®} EP 400 von AKZO) oder auch Siliconpolymere (Silicone Powder X2-1605 von Dow Corning).

Die optionalen teilchenförmigen Feststoffe können sowohl einzeln als auch im Gemisch eingesetzt werden.

Weiterhin ist die Kombination der erfindungsgemäßen Siliciumdioxid-Partikel (d) mit in Wasser Schichtsilikaten oder mit teilchenförmigen organischen Feststoffen bevorzugt.

Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 - 4 Gew.-%, jeweils bezogen auf das Gesamtgewicht der erfindungsgemäßen Zusammensetzung.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, Verdickungsmittel, anfeuchtende und/oder feuchthaltende Substanzen, Fette, Öle, Wachse, Alkohole, Polyole, Polymere, Elektrolyte, organische Lösemittel oder Siliconderivate. Vorteilhafte Konservierungsmittel sind beispielsweise Formaldehydabspalter (wie z. B. DMDM Hydantoin), lodopropylbutylcarbamate, Parabene, Phenoxyethanol, Ethanol, Benzoesäure und dergleichen mehr.

Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter dem Handelsnamen Lipochroman-6 von Lipotec.

Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica. Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epi-catechingallat, (-)-Epigallocatechin, (-)-Epigallocatechingallat. In einer weiteren bevorzugten Ausführungsform enthalten die erfindungsgemäßen Zusammensetzungen mindestens ein Flavonoid oder mindestens einen Flavonoid-reichen Pflanzenextrakt. Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglu-cosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-manno-pyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon. Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 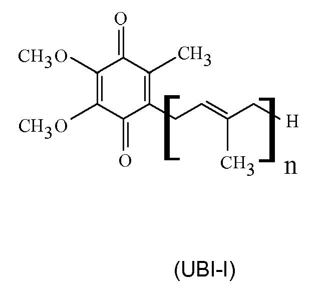 mit n = 6, 7, 8, 9 oder 10.

Besonders bevorzugt ist das Ubichinon der Formel (UBI-I) mit n = 10, auch bekannt als Coenzym Q10. Erfindungsgemäß besonders bevorzugte Zusammensetzungen sind **dadurch gekennzeichnet, dass** sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß besonders bevorzugte Zusammensetzungen sind **dadurch gekennzeichnet, dass** sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II) 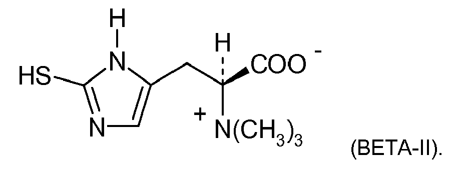 Die Herkunft der eingesetzten natürlichen Betainverbindung kann erfindungsgemäß durchaus synthetisch sein. Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung. Es kann auch von Vorteil sein, beliebige Enantiomerengemische, beispielsweise ein Racemat aus D-und L-Form, zu verwenden.

Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Weitere erfindungsgemäß bevorzugte Zusammensetzungen sind **dadurch gekennzeichnet, dass** sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein. In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversionstemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glu-cosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und

Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn-Salze.

Die erfindungsgemäßen Zusammensetzungen enthalten die Emulgatoren bevorzugt in einer Gesamtmenge von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher **dadurch gekennzeichnet, dass** sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrigviskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. anionische Polymere aus Acrylsäure, Methacrylsäure, Crotonsäure, Maleinsäureanhydrid und 2-Acrylamido-2-methylpropan-sulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere bevorzugte Zusatzstoffe sind Lösungsmittel wie Ethanol, Isopropanol, Ethylenglykol, Propylenglykol, Propylenglykolmonoethylether, Glycerin und Diethylenglykol, Parfümöle, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O Dimethylether, CO₂ und Luft.

In einer weiteren bevorzugten Ausführungsform liegen die erfindungsgemäßen Zusammensetzungen in einem Sprühspender oder Pumpspender verpackt vor.

Die nachfolgenden Beispiele sollen den Gegenstand der vorliegenden Anmeldung verdeutlichen, ohne ihn hierauf einzuschränken. Die Zahlenwerte in den Beispielen bedeuten Gewichtsprozente, bezogen auf das Gesamtgewicht der jeweiligen Zusammensetzungen.

### Testrezepturen (Öl-in-Wasser-Emulsionen)

| | Rezeptur A | Rezeptur B |
|---|---|---|
| Emulgade SE-PF | 8 | 8 |
| Cetiol SN | 5 | 5 |
| Caprylic/Capric Triglycerides | 3,5 | 3,5 |
| Safloröl | 2 | 2 |
| Behenylalkohol | 3 | 3 |
| Silikonöl 350 cs | 0,8 | 0,8 |
| Vitamin E Acetat | 0,5 | 0,5 |
| Controx KS | 0,05 | 0,05 |
| Parsol 1789 | 3 | 3 |
| Parsol SLX | 3 | 3 |
| Propylparaben | 0,2 | 0,2 |
| Hexandiol-1,6 | 5 | 5 |
| Glycerin 86% | 4,5 | 4,5 |
| Sorbit 70% | 2 | 2 |
| Methylparaben | 0,2 | 0,2 |
| Tego Carbomer 140 | 0,5 | 0,5 |
| Na₃-Nitrilotriacetat-wässrig (40%) | 0,1 | 0,1 |
| Talkum | 1,3 | 1,3 |
| Siliciumdioxid-Partikel* | 2 | - |
| Neo Heliopan Hydro | 3 | 3 |
| 2-Amino-2-methylpropanol | 0,99 | 0,99 |
| Natriumhydroxid Perlen | 0,172 | 0,1926 |
| Wasser, vollentsalzt | ad 100 | ad 100 |

| | | |
|---|---|---|
| * nicht organisch oder anorganisch modifiziert, keine organische oder anorganische Beschichtung, zu mehr als 80 Gew.-% sphärische Partikel, zahlenmittlerer Partikeldurchmesser 6 - 8 µm, Ölabsorptionskapazität : 0,9 - 1,3 cm³ flüssiges Paraffin pro Gramm trockenes Siliciumdioxid (DIN 53601), erhalten durch Kieselsäure-Fällung | | |

Die beiden O/W Emulsionen A und B wurden nach Colipa Richtlinie bezüglich des in vivo LSF untersucht. Die Messungen ergaben folgende Ergebnisse für den in vivo LSF:
Rezeptur A: 31,0
Rezeptur B: 15,4

Ferner wurden die sensorischen Eigenschaften der Cremes untersucht. Hierbei konnte für die Creme A im sensorischen Test ein verbessertes Hautgefühl im Vergleich zu Creme B festgestellt werden.

### Weitere erfindungsgemäße Formulierungsbeispiele:

**Wasserhaltige Stifte**

| INCI | Abdeckstift | Foundation Stift | Sonnenschutzstift | Lippenstift |
|---|---|---|---|---|
| Rizinusöl | - | - | - | 3,0 |
| Capryl-/Caprinsäure Triglycerid | 5,0 | 5,0 | 8,0 | 3,0 |
| Octyldodecanol | 5,0 | 5,0 | 8,0 | 6,0 |
| Pentaerythrityl Tetraisostearat | 4,0 | - | 8,0 | - |
| Caprylylcarbonat | - | 5,0 | - | - |
| Isopropylpalmitat | - | - | - | 2,0 |
| Jojobaöl | 1,0 | 1,0 | 1,0 | 1,0 |
| Lanolinöl | - | - | - | 1,0 |
| Simethicone | 0,5 | 0,5 | - | 0,5 |
| PEG-45/ Dodecyl Glycol Copolymer | 3,5 | 2,0 | 2,0 | 2,0 |
| Polyglyceryl-3 Diisostearat | - | 1,5 | 2,0 | 2,4 |
| Bis-Diglyceryl Polyacyladipat-2 | 2,0 | - | - | - |
| Cetearylalkohol | - | - | 1,0 | - |
| Cetylpalmitat | - | - | - | 1,0 |
| C16-36 Alkyl Stearate | 1,0 | 2,0 | 1,0 | - |
| C20-40 Alkyl Stearate | 8,0 | 8,0 | 9,0 | 8,0 |
| Carnaubawachs | 1,5 | 0,5 | 2,0 | 2,0 |
| PVP / Eicosene Copolymer | - | - | 1,0 | 0,2 |
| Eusolex T-AVO | 2,0 | 1,0 | 4,0 | - |
| Ethylhexyl Methoxycinnamate | - | - | 1,0 | - |
| Bis-Ethylhexyloxyphenol methoxyphenyl Triazine | 0,50 | 1,50 | 3,50 | - |
| Butyl Methoxydibenzoylmethane | 3,0 | 3,0 | 3,0 | 2,00 |
| Neo Heliopan AP | - | - | - | 0,75 |
| Ethylhexyl Triazone | 3,50 | - | - | - |
| Polysilicone-15 | 3,0 | 3,0 | 3,0 | 2,00 |
| Diethylhexyl Butamido Triazone | - | 2,00 | 0,75 | |
| 2-Phenylbenzimidazol-5-sulfonsäure | 3,0 | 3,0 | 3,0 | 1,00 |
| Methylene Bis-Benzotriazolyl Tetramethylbutylphenol | - | - | - | 2,50 |
| Ethylhexyl Salicylate | - | 3,50 | - | - |
| Homosalate | 2,00 | - | - | - |
| Diethylhexyl-2,6-naphthalate | - | - | - | 5,50 |
| Siliciumdioxid-Partikel* (siehe oben, Testrezeptur A) | 2,00 | 2,00 | 2,00 | 2,00 |
| BiOCl₃ | - | 3,0 | - | - |
| Bornitrid | - | - | 3,0 | 1,0 |
| Lauroyl Lysin | 0,5 | - | - | - |
| Tospearl 145A | 3,0 | 0,5 | 1,0 | 5,0 |
| Micropearl^{®} M 310 | 6,0 | 3,0 | - | - |
| Interferenzpigmente | - | - | 1,0 | - |
| Titandioxid, Cl 77891 | 5,0 | 3,6 | 1,2 | 4,5 |
| Eisenoxide Cl 77491, 77492, 77499 | 3,6 | 1,8 | 0,8 | 3,2 |
| D&C Rot 7 | - | - | - | 4,5 |
| D&C Rot 6 | - | - | - | 0,2 |
| FD&C Blau 1 | - | - | - | 0,5 |
| D&C Rot 21 | 0,1 | - | - | - |
| FD&C Gelb 6 | - | - | - | 0,2 |
| D&C Rot 34 | - | - | - | 0,5 |
| Citronensäure | - | 0,05 | - | - |
| Glycerin | - | 10 | - | 10 |
| Panthenol | - | - | 1,0 | - |
| Parfum | 1,0 | 1,0 | 1,0 | 1,0 |
| Methylparaben | 1,0 | 1,0 | 1,0 | 1,0 |
| Propylparaben | 1,0 | 1,0 | 1,0 | 1,0 |
| Lipochroman-6 | 0,5 | 0,5 | 0,5 | 0,5 |
| Wasser | ad 100 | ad 100 | ad 100 | ad 100 |

**O/W-Emulsionen**

| Inhaltsstoffe | Foundation | Glanzcreme | Getönte Tagescreme | Foundation |
|---|---|---|---|---|
| PEG-30 Stearat | 1,5 | - | - | - |
| PEG-40 Stearat | - | 2,0 | - | - |
| PEG-100 Stearat | - | - | - | 1,0 |
| Glyceryl Stearat | 0,5 | 1,2 | - | 3,0 |
| Ceteareth-20 | 1,0 | - | - | - |
| Stearinsäure | 2,0 | - | - | - |
| Glyceryl Stearat Citrat | - | - | 3,5 | - |
| Cetyl Alkohol | 0,5 | 2,0 | 0,75 | 1,0 |
| Cetylpalmitat | - | 1,0 | - | - |
| Veegum K = Mg-Al-Silikat | 0,8 | - | - | - |
| Xnthan Gummi | 0,2 | - | - | - |
| Carbomer | - | 0,3 | 0,2 | 0,2 |
| Hydroxyethylcellulose | 0,2 | - | - | - |
| Capryl-/Caprinsäure Triglycerid | - | - | 2,0 | - |
| Dicaprylylether | - | 3,0 | 2,0 | - |
| Dimethicone | 3,0 | 2,0 | - | - |
| Cyclomethicone | - | - | 4,0 | 1,0 |
| C12-C15 Alkyl Benzoat | 2,0 | - | - | - |
| Cetearyloctanoat | 2,0 | - | - | - |
| Squalan | 1,0 | - | - | 1,0 |
| Isopropylpalmitat | 1,0 | - | - | - |
| PPG-15 Stearylether | 2,0 | 2,0 | 3,0 | - |
| Vaseline | - | - | - | 2,0 |
| Hydrierte Kokosglyceride | 2,0 | - | - | 1,0 |
| Hydrierte Polydecen | - | 2,0 | - | - |
| Stearyl Dimethicone | 9,0 | - | - | - |
| Ethylhexyl Methoxycinnamat | - | 4,50 | - | - |
| Bis-Ethylhexyloxyphenol methoxyphenyl Triazine | 1,00 | 2,50 | 3,00 | - |
| Butyl Methoxy Dibenzoylmethane | 2,00 | 2,50 | 3,00 | 2,00 |
| Neo Heliopan AP | - | - | - | 2,00 |
| Ethylhexyl Triazone | - | 1,50 | - | - |
| Octocrylene | 3,00 | - | - | - |
| Diethylhexyl Butamido Triazone | - | - | 3,00 | - |
| Neo Heliopan Hydro | 2,00 | 3,00 | 3,00 | - |
| Methylen Bis-Benzotriazolyl Tetramethylbutylphenol | 2,00 | 1,50 | - | - |
| Polysilicone-15 | 3,00 | 3,00 | 2,00 | 2,00 |
| Diethylhexyl-2,6-naphthalat - | - | - | - | 3,50 |
| Ronasphere LDP | 1,0 | - | 4,0 | - |
| Tospearl 145A | - | 5,0 | - | 3,0 |
| Siliciumdioxid-Partikel* (siehe oben, Testrezeptur A) | 2,00 | 1,50 | 2,00 | 1,50 |
| Eisenoxide Cl 77491, 77492, 77499 | 1,2 | - | 0,8 | 2,6 |
| Titandioxid, Cl 77891 | 3,8 | - | 1,2 | 4,5 |
| Ultramarin, Cl 77007 | 0,5 | - | - | 0,6 |
| Interferenzpigmente | 0,8 | 6,0 | - | - |
| Glycerin | 2,0 | 2,0 | 5,0 | 10,0 |
| Parfum | 1,0 | 1,0 | 1,0 | 1,0 |
| Methylparaben | 1,0 | 1,0 | 1,0 | 1,0 |
| Propylparaben | 1,0 | 1,0 | 1,0 | 1,0 |
| Lipochroman-6 | 0,5 | 0,5 | 0,5 | 0,5 |
| Wasser | ad 100 | ad 100 | ad 100 | ad 100 |

**O/W-Sonnenschutz-Emulsionen**

| | 1 | 2 | 3 | 4 | 5 |
|---|---|---|---|---|---|
| Glycerylstearat Citrat | 2.00 | 1.50 | 3.00 | - | - |
| Glycerylstearat | - | - | - | 2.00 | 2.00 |
| PEG-40 Stearat | | - | - | - | 2.00 |
| Stearinsäure | - | - | - | 3.00 | - |
| Cetyl Phosphat | - | - | - | 0.50 | - |
| Caprylic/Capric Triglyceride | 0.50 | - | 1.00 | - | 1.50 |
| Cetearyl Alkohol | 3.00 | 1.00 | - | - | - |
| Cetyl Alkohol | - | - | 3.00 | - | 2.00 |
| Stearyl Alkohol | - | 1.00 | - | - | 2.00 |
| Lanolin Alkohol | - | 0.50 | - | 0.50 | - |
| Myristyl Alkohol | - | - | - | 3.00 | - |
| Octyldodecanol | 1.50 | - | - | - | - |
| C18-36 Säure Triglycerid | 1.50 | 2.00 | - | - | - |
| Mineralöl | - | - | - | 2.00 | - |
| Ethylhexyl Methoxycinnamat | - | 4.00 | - | - | - |
| Bis-Ethylhexyloxyphenol | 2.00 | - | 1.50 | - | 1.00 |
| Butyl Methoxydibenzoylmethane | 2.00 | 2.00 | 2.00 | 1.50 | 3.00 |
| Neo Heliopan AP | - | 0.50 | - | 2.00 | - |
| Ethylhexyl Triazone | 3.00 | - | 2.00 | - | - |
| 4-Methylbenzyliden Camphor | 2.00 | - | - | 4.00 | - |
| Octocrylene | 5.00 | - | 7.50 | 6.00 | 9.00 |
| Diethylhexyl Butamido Triazone | 1.00 | 2.00 | - | 0.05 | - |
| Neo Heliopan Hydro | 2.00 | 3,0 | 1.00 | ,00 | 3,00 |
| Methylen Bis-Benzotriazolyl Tetramethylbutylphenol | - | 2.00 | - | 2.50 | 1.00 |
| Drometrizole Trisiloxane | - | - | 1.50 | - | - |
| Mexoryl SX | - | 1.50 | - | - | 0.50 |
| Polysilicone-15 | 3.00 | 3.00 | 3.00 | 4.00 | 5.00 |
| Ethylhexyl Salicylate | - | 2.00 | 4.00 | - | - |
| Homosalate | 3.00 | - | - | - | 5.00 |
| Diethylhexyl-2,6-naphthalat | 2.00 | - | 5.50 | - | - |
| Micro Titanium Dioxide MT-100Z | 2,0 | - | - | 1,5 | - |
| Siliciumdioxid-Partikel* (siehe oben, Testrezeptur A) | 2,0 | 1.50 | 2,0 | 2,0 | 2,0 |
| C12-C15 Alkyl Benzoate | 2.00 | - | 1.50 | 2.50 | 0.80 |
| Dicaprylyl Carbonate | - | 3.00 | - | - | - |
| Butylenglycol Dicaprylat/Dicaprat | 5.00 | - | - | 6.50 | 3.00 |
| Butylenglycol | - | 7.50 | - | 4.50 | 5.00 |
| Carbomer | 0.30 | 0.10 | 0.20 | - | 0.35 |
| Tocopherylacetat | 0.30 | - | 0.50 | - | 0.80 |
| Dicaprylyl Ether | 2.50 | 0.50 | - | 2.00 | 1.50 |
| Polyurethane | - | 1.00 | - | - | - |
| PVP/Hexadecene Copolymer | 1.00 | - | 0.80 | 1.00 | 0,50 |
| Xanthan Gum | 0.20 | - | 0.40 | - | 0.30 |
| Dimethicone | 2.00 | - | - | 1.00 | - |
| Cyclomethicone | - | 2.50 | - | - | - |
| Glycerin | 5.00 | - | 7.50 | - | 2.00 |
| Distärke Phosphat | 2,00 | - | - | - | - |
| Tospearl 145A | - | - | 6,00 | - | 2,00 |
| Ronasphere LDP | 2,00 | 10,00 | - | - | - |
| Eisenoxide Cl 77491, 77492, 77499 | - | - | - | 2,4 | - |
| Titandioxid, Cl 77891 | - | - | - | 3,6 | - |
| Interferenz Pigmente | - | - | - | - | 5,00 |
| DMDM Hydantoin | - | - | 0.20 | - | 0.40 |
| Propylparaben | - | 0.40 | - | 0.25 | - |
| Methylparaben | 0.60 | - | - | 0.30 | - |
| Trinatrium EDTA | 1.00 | - | 1.00 | 1.00 | 1.00 |
| Phenoxyethanol | 0.15 | - | 0.30 | - | 0.40 |
| Parfüm | 0.20 | 0.20 | 0.20 | 0.20 | 0.20 |
| Wasser | ad 100 | ad 100 | ad 100 | ad 100 | ad 100 |

**W/O-Emulsionen**

| | 1 | 2 | 3 |
|---|---|---|---|
| Triglycerindüsostearat | 1,0 | - | - |
| Diglycerindipolyhydroxystearate | 3,0 | - | - |
| PEG-30 Dipolyhydroxystearate | - | 3,0 | - |
| CETYL PEG/PPG-10/1 DIMETHICONE | - | - | 4,5 |
| Paraffinöl | 12,5 | 10,0 | - |
| Vaseline | 8,0 | 6,0 | - |
| Softisan 138 | 2,0 | 1,0 | - |
| Decyloleat | 0,5 | 0,75 | - |
| Octyldodecanol | 0,5 | 1,0 | - |
| Aluminiumstearat | 0,4 | 0, 3 | - |
| Magnesiumstearat | 0,1 | 0,05 | - |
| hydriertes Rizinusöl | 0,5 | 0,75 | - |
| Cyclomethicone | 1,0 | 1,0 | 24,0 |
| Phenyltrimethicone | - | - | 2,0 |
| hydriertes Polyisobuten | 0,5 | 0,3 | - |
| Capryl/Caprinsäuretriglycerid | 3,0 | 5,0 | - |
| Magnesiumsulfat | 0,5 | 0, 6 | 1,5 |
| Glycerin | 3,0 | 5,0 | 10,0 |
| Ethylhexyl Methoxycinnamate | - | - | 5,0 |
| Butyl Methoxydibenzoylmethan | 1,50 | 2,20 | 3,0 |
| Neo Heliopan AP | 0,50 | - | 2,00 |
| Ethylhexyl Triazone | - | - | 2,00 |
| Polysilicone-15 | 4,00 | 2,00 | 3,00 |
| Diethylhexyl Butamido Triazone | - | 1,50 | - |
| 2-Phenylbenzimidazol-5-sulfonsäure-Argininsalz | 3,0 | 3,0 | 2,0 |
| Methylen3 Bis-Benzotriazolyl Tetramethylbutylphenol | - | 1,00 | 1,50 |
| Zinkoxid NDM | 4,50 | - | 3,6 |
| Titandioxid, Cl 77891 | 3,6 | 4,0 | 3,6 |
| Siliciumdioxid-Partikel* (siehe oben, Testrezeptur A) | 2,2 | 2,2 | 2,2 |
| Quaternium-18 Hectorit | - | - | 0,2 |
| Propylencarbonat | - | - | 0,05 |
| Tospearl 145A | - | - | 6,00 |
| Ronasphere LDP | 2,00 | 10,00 | - |
| Nylon-12 | - | 2,0 | 10,0 |
| Parfum | 1,0 | 1,0 | 1,0 |
| Methylparaben | 0,5 | 0,5 | 0,5 |
| Propylparaben | 0,3 | 0,3 | 0,3 |
| Wasser | ad 100 | ad 100 | ad 100 |

### Liste der verwendeten Rohstoffe

| Handelsname | INCl | Lieferant/Hersteller |
|---|---|---|
| Emulgade SE-PF | Glyceryl Stearate, Ceteareth-20, Ceteareth-12, Cetearyl Alcohol, Cetyl Palmitate | Cognis |
| Cetiol SN | Cetearyl isononanoate | Cognis |
| Controx KS | Tocopherol, Hydrogenated Palm Glycerides Citrate | Cognis |
| Parsol 1789 | Butyl Methoxydibenzoylmethane | DSM |
| Parsol SLX | Polysilicone-15 | DSM |
| Tego Carbomer 140 | Carbomer | Goldschmidt |
| Neo Heliopan Hydro | Phenylbenzimidazol sulfonic acid | Symrise |
| Eusolex T-AVO | Titanium dioxide, Silica | Merck KGaA |
| Ronasphere LDP | Silica, Cl 77891 (Titanium dioxide), Cl 77491 (Iron Oxides) | Merck KGaA |
| Neo Heliopan AP | Disodium Phenyl Dibenzimidazol tetrasulfonate | Symrise |
| Handelsname | INCl | Lieferant/Hersteller |
| Micropearl^{®} M 310 | Methyl Methacrylate Crosspolymer | Seppic |
| Tospearl 145A | Polymethylsilsesquioxane | Momentive Performance Materials |
| Lipochroman-6 | Dimethylmethoxy Chromanol | Lipotec S.A. |
| Micro Titanium Dioxide MT-100Z | Titanium Dioxide, Aluminum Hydroxide, Stearic Acid | Tayca Corporation |
| Lameform TGI | Polyglyceryl-3 Düsostearate | Cognis |
| Dehymuls PGPH | Polyglyceryl-2 Dipolyhydroxystearate | Cognis |
| Softisan 138 | Hydrogenated Coco-Glycerides | Sasol |
| Mexoryl SX | Terephthalylidene Dicamphor Sulfonic Acid | Chimex |

## Patentansprüche

1. Kosmetische Zusammensetzung, enthaltend
a) mindestens einen öllöslichen UV-Filter, umfassend mindestens ein Alkyl-und/oder Alkoxy-substituiertes Dibenzoylmethanderivat,
b) Polysilicone-15,
c) mindestens einen wasserlöslichen UV-Filter, umfassend ein Derivat der Benzimidazolsulfonsäure,
d) 0,2 - 5 Gew.-% Siliciumdioxid-Partikel, die nicht organisch oder anorganisch modifiziert sind und keine organische oder anorganische Beschichtung aufweisen.

2. Kosmetische Zusammensetzung gemäß Anspruch 1, **dadurch gekennzeichnet, dass** die Siliciumdioxid-Partikel zu mehr als 80 Gew.-% sphärische Partikel umfassen.

3. Kosmetische Zusammensetzung gemäß Anspruch 1 oder 2, **dadurch gekennzeichnet, dass** die Siliciumdioxid-Partikel einen zahlenmittleren Partikeldurchmesser im Bereich von 2 - 15 µm aufweisen.

4. Kosmetische Zusammensetzung gemäß Anspruch 1, 2 oder 3, **dadurch gekennzeichnet, dass** die Siliciumdioxid-Partikel einen zahlenmittleren Partikeldurchmesser im Bereich von 5 - 10 µm aufweisen.

5. Kosmetische Zusammensetzung gemäß Anspruch 1, 2, 3 oder 4, **dadurch gekennzeichnet, dass** die Siliciumdioxid-Partikel eine Ölabsorptionskapazität von 0,7 - 1,5 cm³ flüssiges Paraffin pro Gramm trockenes Siliciumdioxid, aufweisen, gemessen nach DIN 53601.

6. Kosmetische Zusammensetzung gemäß Anspruch 1, 2, 3, 4 oder 5, **dadurch gekennzeichnet, dass** die Siliciumdioxid-Partikel durch Kieselsäure-Fällung erhältlich sind.

7. Kosmetische Zusammensetzung gemäß Anspruch 1, 2, 3, 4, 5 oder 6, **dadurch gekennzeichnet, dass** der mindestens eine UV-Filter a) ausgewählt ist aus 4-(tert.-Butyl)-4'-methoxydibenzoylmethan.

8. Kosmetische Zusammensetzung gemäß Anspruch 1, 2, 3, 4, 5, 6 oder 7, **dadurch gekennzeichnet, dass** der mindestens eine UV-Filter c) ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolammonium-Salzen, insbesondere aus Phenylbenzimidazolsulfonsäure und dem Phenylen-1,4-bis-(2-benzimidazyl)-3,3'-5,5'-tetrasulfonsäure-bis-natriumsalz.

9. Kosmetische Zusammensetzung gemäß Anspruch 1, 2, 3, 4, 5, 6, 7 oder 8, **dadurch gekennzeichnet, dass** der mindestens eine UV-Filter c) ausgewählt ist aus den Salzen der 2-Phenylbenzimidazol-5-sulfonsäure mit basischen Aminosäuren, insbesondere mit Lysin, Arginin und/oder Histidin, wobei Arginin besonders bevorzugt ist.

10. Kosmetische Zusammensetzung gemäß Anspruch 1, 2, 3, 4, 5, 6, 7, 8 oder 9, **dadurch gekennzeichnet, dass** der mindestens eine UV-Filter c) ausgewählt ist aus den Salzen der Phenylen-1,4-bis-(2-benzimidazyl)-3,3'-5,5'-tetrasulfonsäure mit basischen Aminosäuren, insbesondere mit Lysin, Arginin und/oder Histidin, wobei Arginin besonders bevorzugt ist.

11. Kosmetische Zusammensetzung gemäß Anspruch 1, 2, 3, 4, 5, 6, 7, 8, 9 oder 10, **dadurch gekennzeichnet, dass** 4-(tert.-Butyl)-4'-methoxydibenzoylmethan, Polysilicone-15 und 2-Phenylbenzimidazol-5-sulfonsäure enthalten sind.

12. Kosmetische Zusammensetzung gemäß Anspruch 1, 2, 3, 4, 5, 6, 7, 8, 9, 10 oder 11, **dadurch gekennzeichnet, dass** 4-(tert.-Butyl)-4'-methoxydibenzoylmethan, Polysilicone-15 und 2-Phenylbenzimidazol-5-sulfonsäure enthalten sind.

13. Kosmetische Zusammensetzung gemäß Anspruch 1, 2, 3, 4, 5, 6, 7, 8, 9, 10, 11 oder 12, **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5) enthalten sind.

14. Kosmetische Zusammensetzung gemäß Anspruch 1, 2, 3, 4, 5, 6, 7, 8, 9, 10, 11, 12 oder 13, **dadurch gekennzeichnet, dass** die UV-Filter a), b) und c) in einem Gewichtsverhältnis zueinander von a) : b) : c) wie (0,9 - 1,2) : (0,9 - 1,2) : (0,9 - 1,2), bevorzugt wie (1 -1,1):(1 -1,1):(1 -1,1), enthalten sind.
